# EUROPEAN PATENT SPECIFICATION

(11) **EP 3 927 251 B1**
(45) Date of publication and mention of the grant of the patent: **07.06.2023**
(21) Application number: 20705960.1
(22) Date of filing: 18.02.2020
(51) Int. Cl.: A61B 17/132

(54) **MEDICAL DEVICE FOR BLOOD VESSEL COMPRESSION**
MEDIZINISCHE VORRICHTUNG ZUR KOMPRESSION VON BLUTGEFÄSSEN
DISPOSITIF MÉDICAL POUR LA COMPRESSION DE VAISSEAUX SANGUINS

(30) Priority: 19.02.2019 PL 42896419
(43) Date of publication of application: 29.12.2021
(73) Proprietor: Simplicardiac S.A., 02-593 Warszawa (PL)
(72) Inventor: KRUK, Mariusz, 05-070 Poland (PL)
(74) Representative: Patpol Kancelaria Patentowa Sp. z o.o.
(86) International application number: PCT/EP2020/054189
(87) International publication number: WO 2020/169573

(56) References cited:
- WO-A2-2013/060883
- US-A1- 2010 280 541
- US-A1- 2019 090 886

## Description

### TECHNICAL FIELD

Present disclosure relates to a medical device for blood vessels compression that is applied to a limb of a patient in order to achieve local haemostasis. In particular, the device of the disclosure is used in invasive cardiology and radiology to compress a blood vessel upon removal of a vascular sheath from a patient's blood vessel after heart catheterization, coronary angiography or other intravascular procedures and interventions that require a vascular access port.

### PRIOR ART

Many different blood vessel compression devices are known in the prior art. For example, WO2013060883 discloses an arterial compression band having a tube-like shaped body with bevelled endings and a holding element in a form of a strap that is attached to the outside surface of the body. This medical device is applied at a bleeding vessel site, in particular, the bleeding site of an artery (radial or ulnar) from which a catheter or cannula has been removed, by pressing manually the body against the bleeding vessel site, followed by immobilizing the body on the patient's limb by applying the holding element around the patient's limb. The device is fixed to the patient's limb by the fastening means, such as a hook and loop fastener, which can be located on the holding element. The device disclosed therein is quite simple in manufacturing and for application to a patient's limb. Most of all, it provides manual control of the blood vessel compression upon placing the device on a patient's limb, i.e. a medical personnel (for example a physician or a nurse) can apply the body to the site of the catheter or cannula entry to the blood vessel, while gripping on the inside of the hollow body, remove the catheter or cannula and, at the same time, exert the necessary compression to the blood vessel, and fix the body to the patient's limb by applying the strap around the patient's limb and over the body of the device while still holding the inside of the hollow body.

However, once applied on a patient's limb the device is prone to accidental displacement, either by tipping over or by shifting or sliding. This influences the compression force exerted on blood vessel or even the compression application site on the limb after the device was applied, making the device less effective at its primary purpose. Moreover, after the device is fixed to the patient's limb, the device does not provide a sufficient control of the compression level, especially when the compression needs to be gradually decreased after the device is placed on the patient. The compression is controlled only by strap positioning or by the means of adjusting the cross-section dimensions or shape of the body. However, in the first case the adjustment of the strap is required, which is very inconvenient and requires the medical staff to unfasten the holding means and fasten it again in a new position, risking device displacement that may result in bleeding. In the latter case, the construction of the body becomes very complex and the level of compression adjustment is also very limited.

In order to ensure sufficient compression control in compression haemostatic devices, screw-and-nut systems can be used to exert pressure onto a patient's limb. In US20100280541, a radial artery compression device is disclosed that includes a rotatable member and a compression pad adapted in such a way that rotation of the rotatable member does not affect the rotational orientation of the compression pad. The rotation of the rotatable member results in extension or retraction of the compression pad relative to the body of the radial artery compression device, thus increasing or decreasing, respectively, the compression on a patient's radial artery. Therefore, in such devices the screw-and-nut system drives the compression pad directly, thus enabling precise compression control. A similar device is described in US20120191127. However, in this case the screw-and-nut system can also be located outside the diameter of the compression pad, which is attached to the base of the device. In this case, the compression level is controlled by deflection of the entire base.

Although, the above-described devices provide more precise control of the compression level, they are not very easy to apply. They do not provide the manual control upon application as the device described in WO2013060883. Even though the rotatable member is provided with a recess that can receive a finger or a thumb of a medical personnel, who controls the pressure of the device upon placing the device of a patient's limb, this recess is not very convenient for gripping, as it can only be accessed from the top of the rotatable member. Moreover, although said recess is provided with a transparent floor, the view of the catheter or cannula entry site is still significantly blocked by the screw-and-nut system elements. Finally, in both devices of US20100280541 and US20120191127 the holding means or base is in direct contact with the radial side of the patient's forearm. Such a design not only provides discomfort to a patient when the device is applied, but also affects the blood flow in neighbouring blood vessels, in particular arm veins or the ulnar artery, because some compression is unintentionally applied through the body and the straps. Devices for the compression of a selected blood vessel, for example, a more accurate compression in which a radial artery is compressed while maintaining the blood flow in the neighbouring blood vessels would be useful but is not apparent from the prior art.

It is asserted that there remains a need for a device which can be securely fixed in position on a limb and which provides specific and accurate blood vessel compression. Moreover, it would be advantageous if the device is able to precisely control that compression without compromising on the simplicity of its construction or ergonomic design.

### SUMMARY OF THE INVENTION

The invention provides a vessel compression device for a limb, which comprises:
- a hollow body having a generally tubular space extending in a longitudinal direction, said tubular space forming an internal substantially annular surface and a longitudinal base said base being accessible by the generally tubular space;
- a compression area located at an external surface of the base;
- a compression control mechanism for regulating compression applied by the device, wherein the mechanism is located on the body distal to and opposite the compression area and the base;
- a strap for securing the device to the limb and effecting compression thereon, the strap being attached to body at the base and releasably attachable with and adjustable by the compression control mechanism without blocking the tubular space;
- a support for stabilising the device, the support being located adjacent the base and extending in a longitudinal direction parallel therewith.

When applied to an injured vessel, embodiments of the medical device disclosed herein provide blood vessel compression, in particular radial compression, for the purpose of establishing haemostasis. The blood vessel compression device of the invention is ergonomically designed, so that it is easily attached to the patient limb and very convenient to use by a medical personnel. When the device of the invention is applied to the patient's limb it is stable (i.e. it is not prone to sliding or tipping) and provides a sufficient compression control. The device of the invention also provides a greater comfort to a patient, as the compression on the radial side of a patient's forearm is essentially limited to the catheter or cannula blood vessel entry site. The design of the device of the invention also prevents compression of the blood vessels other than the blood vessel with the vascular access port. The device of the invention is constructed in such a way that its elements are easy to manufacture by plastic injection moulding. While designing the device, the inventors took under consideration the shape of injection moulds, to ensure that they have a simple construction and that the elements produced by injection moulding could be easily retrieved from moulds.

In a preferred embodiment, the compression control mechanism of the device of the invention is based on a screw-and-nut system. Also preferably the strap, which, in embodiments, is attached to a hollow body at the vicinity of the compression area (i.e. on the opposite side of the hollow body relative to the compression control mechanism), upon application of the device on the patient's limb is guided across and over the compression control mechanism, which regulates the compression of the device by adjusting the tension of the strap.

Also in this embodiment the hollow body has a cylindrical-like shape with bevelled endings. More preferably, the tubular space inside the hollow body is formed by a through-hole. In this embodiment, i.e. whenever the tubular space inside the hollow body can be accessed from both sides, the vessel compression device according to the invention can be used on both forearms of the patient and to compress different vessels.

In embodiments of the invention, the compression control mechanism comprises a knob and a tightener, wherein the knob is fitted over an externally-threaded element of the hollow body, which is located on the opposite side of the hollow body, relative to the compression area, and supports the tightener. When the device of the invention is attached to the patient's limb and the knob is turned, it moves along the externally-threaded element of the hollow body and changes the vertical position of the tightener. As the tightener is moved in the vertical position, it does not turn around horizontally, providing a support for the strap. As the knob and the tightener move away from the middle of the hollow body (i.e. away from the compression area), the tightener presses upon the strap and increases its tension, thus increasing the compression of the device against patient's limb. When the knob is turned in the opposite direction, it moves along the externally-threaded element of the hollow body towards the middle of the hollow body (i.e. towards the compression area). The tightener follows the movement of the knob, thus decreasing the tension of the strap and the compression of the hollow body against the patient's limb.

The compression control mechanism, which adjusts the tension of the strap, allows for precise control of the compression of the device against the patient's limb. Also, due to the fact that the strap is guided around the hollow body, it does not compress the radial side of the patients forearm, thus eliminating the compression of other blood vessels, and, at the same time it does not block the access to the tubular space of the hollow body, thus allowing a comfortable grip of the device of the invention by pressing the compression area of the hollow body with a finger or a thumb of a medical personnel received in said tubular space of the hollow body. The hollow body, the compression control mechanism and the strap, are preferably made of transparent material and do not block the view of the blood vessel compression area. This enables visual control of the catheter or cannula vessel entry site upon or after removal thereof. Therefore, it is possible to determine if the haemostasis is achieved or to detect the bleeding from the injured vessel.

In one preferred embodiment, the stabilizing support protrudes from the hollow body in the proximity of its compression area. Additionally, in the preferred embodiment, the support for stabilizing the body is in a form of a bent fin, whose edge opposite to the edge attached to the hollow body is in the same plane as the compression area of the hollow body, thus providing the support for the hollow body, whereas the remaining part of the support is raised above the plane of the compression area. Such a shape of the stabilizing support minimalizes the contact of the device of the invention with the patient's limb, thus increasing the patient's comfort and decreasing the compression of the device on the area of the patient's limb other than the site of the punctured blood vessel. That way the compression of blood vessels other than that providing the vascular access point is minimalized. The device of the invention is therefore designed to provide precise compression control onto a selected blood vessel, while maintaining the blood flow in neighbouring blood vessels unaffected.

If the device is used to compress the radial artery, the ulnar artery is not compressed as the stabilizing support is raised above the surface of the patient's forearm region, which corresponds to the position of the ulnar artery. Accordingly, if the device is used to compress the ulnar artery (i.e. it is applied to the limb in the opposite direction), the radial artery is not compressed. Most of all, due to the device design, in particular the design of the stabilizing support, whose contact with the patient's limb is limited, the device does not compress the neighbouring veins. Therefore, the venous outflow from the hand is not hindered by the compression of the device on patient's limb and venostasis is avoided. This not only increases patient comfort, but also reduces the risk of side effects associated with catheterization and compression of the limb.

Even though only the edge of the stabilizing support opposite to the edge connected to the hollow body is in contact with the patient's forearm, the desired stability to the entire device of the invention is provided. The stabilizing support prevents the displacement of the device of the invention by tipping over or sliding, while it is attached to the patient's limb. In the most preferred embodiment, the stabilizing support is in a form of a bent fin having a wave-type cross section, wherein the edge of the support opposite to the edge connected to the hollow body is provided with a supporting foot. This supporting foot increases the patient's comfort, when the device of the invention is attached to the patient's limb.

In the preferred embodiment the strap of the device of the invention is made of anti-skid material. Preferably, the strap is made of a material that requires no additional fastening means to hold the device of the invention of the patient's limb. In particular, one surface of the straps - the outer surface, adheres to the opposite surface of the strap - the inner surface, when the strap is secured around the patient's limb and the outer and inner surface of the strap come in contact. In an alternative embodiment, the strap can be provided with additional fastening means for holding the device in a desirable position on the patient's limb selected from a group comprising an adhesive (for example, in a form of a locking sticker), tightening buckles, hooks, snap fasteners (for examples, orifices and the corresponding lugs) or hook and loop fasteners, such as Velcro°. In some preferred embodiments the fastening means comprises two parts situated on the opposite surfaces of the strap, which come into contact when the device of the invention is attached to the patient's limb. In the preferred embodiment the fastening means are provided at the free end of the strap, i.e. the end of the strap opposite to the strap end connected to the base of the hollow body.

In embodiments, the device of the invention is equipped with a clicking mechanism, which indicates the turn of the knob on the externally-threaded element. That way an acoustic control of the compression adjustment is possible. The clicking mechanism according to the preferred embodiment comprises a ring spring, which is fixed within the tightener, wherein said ring spring comprises downward protrusions that engage with uniformly spaced cogs located in the vicinity of the knob rim closest to the tightener. That way, whenever the knob is turned the protrusion of the ring spring is displaced from one space between the cogs to another, wherein upon the displacement it engages with the cog and produces a clicking sound. The system consisting of the ring spring with protrusions that engage with the elements on the knob is very space efficient. Therefore, the clicking functionality of the device of the invention can be introduced without increasing the size of the entire device of the invention. Moreover, the design of the clicking mechanism renders its manufacturing method quite simple, since it does not require any complex injection moulding forms.

### BRIEF DESCRIPTION OF THE DRAWINGS

The present invention will be described in a greater detail herein below in reference to a drawing wherein:
Fig. 1a presents a perspective view of the vessel compression device according to the preferred embodiment of the present invention is shown in the folded form;
Fig. 1b presents a side view of the device presented in Fig. 1a in an unfolded form;
Fig. 2a presents an exploded view of the vessel compression device of the invention from Fig. 1a and 1b;
Fig. 2b and 2c presents enlarged parts of the exploded view A and B, respectively, from Fig. 2a;
Fig. 3 presents the hollow body of the vessel compression device of the invention in a perspective view (Fig. 3a), in a front view (Fig. 3b), side views (Fig. 3c) and bottom view (Fig. 3d);
Fig. 4 presents a tightener of the vessel compression device of the invention in a perspective view from the bottom (Fig. 4a), in a bottom view with a ring spring (Fig. 4b), in a top view (Fig. 4c) and in the side views (Fig. 4d and Fig. 4e);
Fig. 5 presents a knob of the compression device of the invention in a perspective view from the bottom (Fig. 4a) and from the top (Fig. 5b), in a top view (Fig. 5c) and a cross-section along A-A line of the knob presented in Fig. 5c (Fig. 5d);
Fig. 6 presents a perspective and top views of the ring spring in the vessel compression device of the invention; and
Fig. 7 presents a bottom view of a strap of the vessel compression device of the invention.

### DETAILED DESCRIPTION

The present invention relates to a vessel compression device 1.

Whenever a reference is herein made to a term "bottom" or "below" or "underneath" with respect to the device 1 or an element thereof, it means a part of the device 1 proximal to the patient and limb desired to be compressed, when the device 1 is properly attached to said limb. The term "downwards" also means in a direction towards the patient's limb.

The term "top" or "above" as used herein in reference to the device 1 or an element thereof, refers to the part of the device 1, which is located distal from the patient's limb, when the device 1 is properly attached to said limb. The term "upwards" refers to a distal direction from the patient's limb.

The device 1 of the invention has been designed to apply proper compression forces in the specific area of the limb in order to prevent bleeding from an injured blood vessel. At the same time, the device 1 does not apply forces to other major blood vessels in a patient's limb.

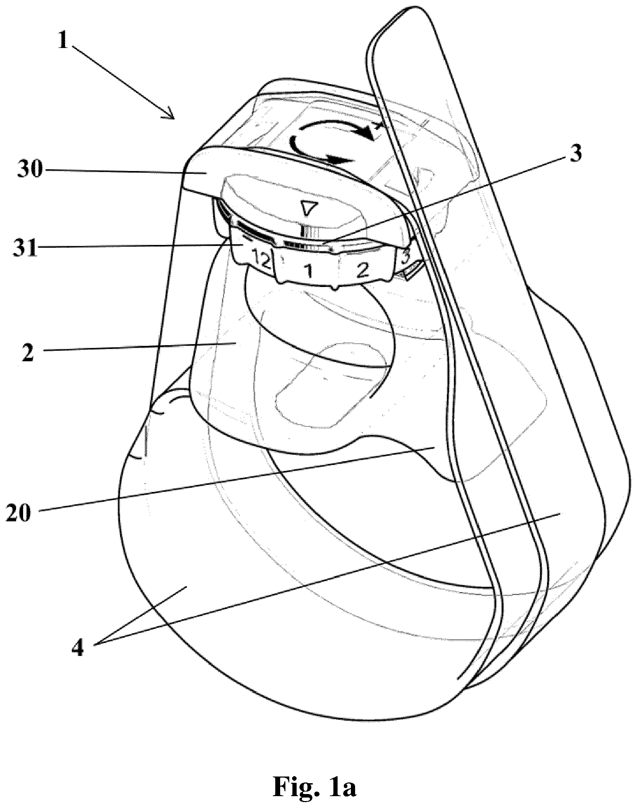

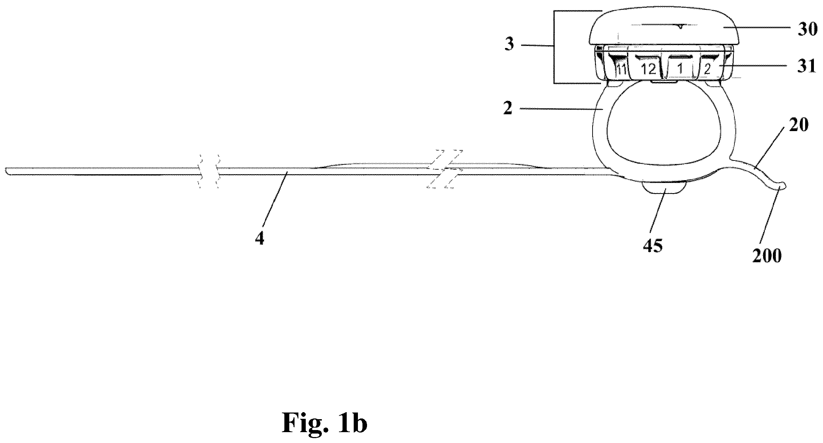

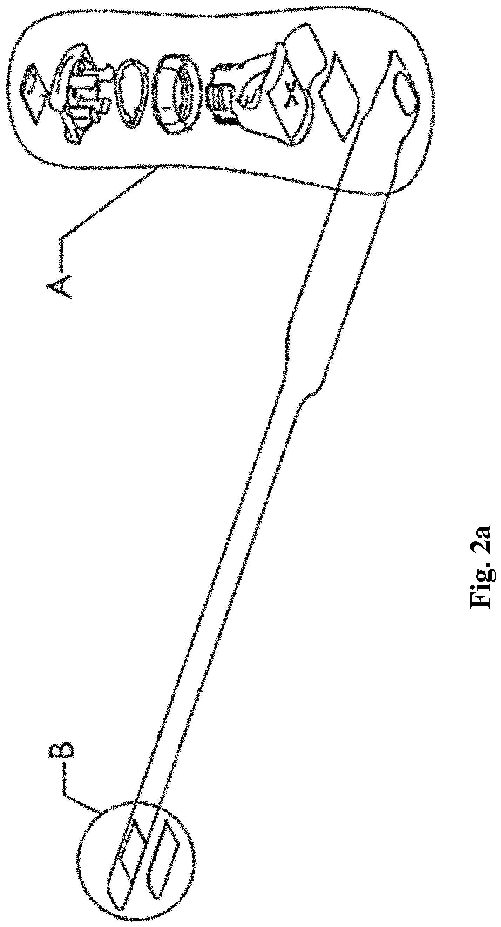

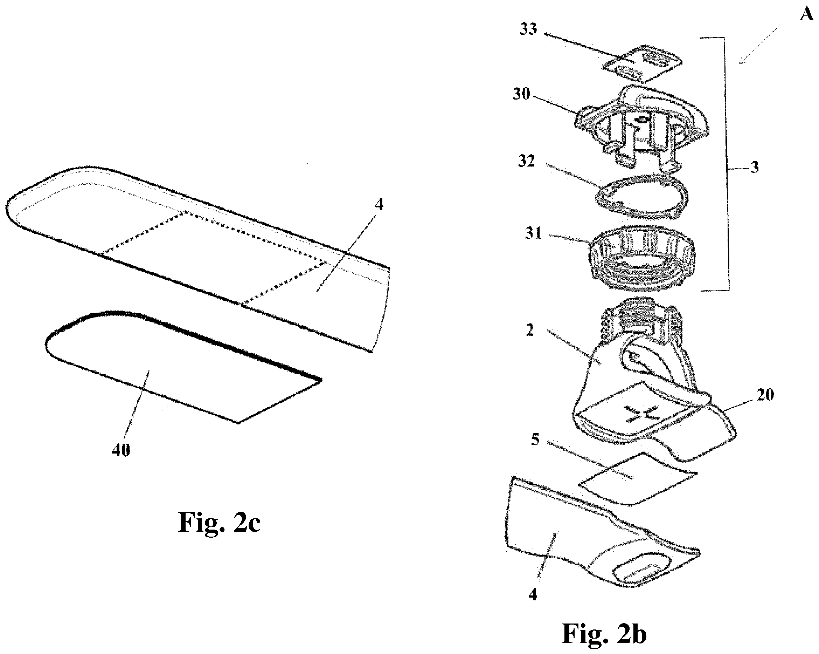

As presented in Figs. 1a and 1b and Figs. 2a, 2b and 2c, the device 1 comprises four main elements: a hollow body 2, a compression control mechanism 3 comprising a tightener 30 and a knob 31, and a strap 4. The hollow body 2 forms a central element of the device 1. The strap 4 is securely attached to the hollow body 2, in particular to a base 26 of the hollow body 2. This ensures that the strap 4 remains attached to the hollow body 2 during regular use, when it is subjected to significant forces. In this embodiment the strap 4 is attached to the compression area 24 by means of an adhesive 5 and the contact area between the strap 4 and the hollow body 2 corresponds to the entire compression area 24 of the hollow body 2. This large contact area not only ensures that the strap 4 is well secured to the hollow body 2, but also provides additional stability to the device 1 when the device is attached to the patient's limb, in particular prevents the device 1 from sliding sideways when the device is applied or when the compression forces are adjusted.

On the opposite side to the strap 4 attachment site, i.e. at the top, the device 1 is provided with a tightener 30 and a knob 31, both of which are in a bolt-nut movable connection with hollow body 2, in particular with the externally-threaded element 21 of the hollow body 2. The tightener 30 is supported by the knob 31, which is located underneath of the tightener 30. After the device 1 is assembled, the tightener 30 and the knob 31 move together relative to the hollow body 2 along the externally-threaded element 21 of the hollow body 2. The movement of the tightener 30 relative the hollow body 2 provides control of the compression of the device 1 onto the patient's limb.

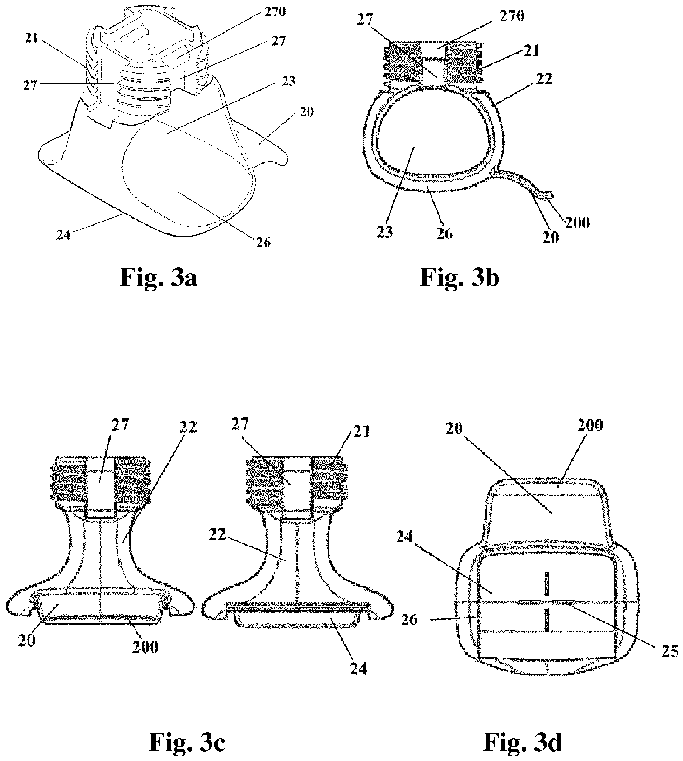

As shown in Figs. 3a-d the hollow body 2 is formed as an integral element. In this embodiment, the hollow body 2 is in a form of a short tube with a cross-section of a flattened circle. In particular, the base 26 of the hollow body 2 flattened to provide a better contact with the patient's limb. However, it can be formed in any other shape that will be configured to provide a medical personnel with a suitable access to the compression area 24 via the base 26 of the hollow body 2 (i.e. the tubular space 23 within the body 2, where the medical personnel can insert his or her finger in order to apply the force onto the bottom 26 of the hollow body 2, so that upon application of the device 1 to the patient's limb the medical personnel can apply force to injured vessel manually). In embodiments, the hollow body 2 is symmetrical along its long axis, thus rendering the device of the invention operational on both hands. Moreover, the selected shape of the body 2 needs to accommodate the tightener 30 and the knob 31 placed above the tubular space 23 within the hollow body 2. For example, the body 2 can be in a shape of a half-pipe, whose walls are connected by an externally-threaded element 21 that receives the tightener 30 and knob 31. In the presently described preferred embodiment, the tube that forms the central part of the hollow body 2 has a longitudinal base 26, which is directed towards a patient's limb, when the device 1 is applied properly. The base 26 is connected with the externally-threaded element 11 by side walls 22. The base 26 of the tube is wider than the side walls 22 of the tube and has a larger surface area than the side walls 22. The outer surface of the base 26 of the hollow body 2 provides a compression area 24, which is configured to be in contact with the patient's limb when the device 1 is applied. In the present embodiment, the compression area 24 is covered with an adhesive 5 that connects the strap 4 to the compression area 24. Therefore, the compression area 24 is in contact with the patient's limb through the strap 4 and adhesive 5. Moreover, the compression area 24 preferably comprises a mark 25 that facilitates the positioning of the device 1 on patent's limb. When applied to a patient's limb, the positioning mark 25 should be placed at the site of the vascular access port. In the preferred embodiment the hollow body 2 together with its integral elements is formed of transparent plastic material. Use of the transparent material ensures a visual control of the catheter or cannula entry site on the patient's limb. In this embodiment, the body 2 is made of polycarbonate by injection moulding. Additionally, the edges of the hollow body 2 are blunt to increase the comfort of the patient.

The hollow body 2, in embodiments, is provided with a support 20, which may be an integral part of the hollow body 2. The support 20 extends from a side of the hollow body 2 (i.e. from the side wall 22 of the hollow body 2 near the base 26 of the hollow body 2), and away from the compression area 24.

In the preferred embodiment, the support 20 is in a form of a bent fin having a wave-type cross section. Initially, as it extends away from the hollow body 2, the bent fin of the support 20 is directed upwards to the inflection point where its direction changes towards the plane of the compression area 24. The support 20 does not extend beyond the plane of the base 26 of the hollow body 2. Moreover, at its free end (i.e. opposite to the site where the support 20 is connected to the side wall 22 of the hollow body 2), the support 20 is equipped with a supporting foot 200. When the device is applied to the patient's limb, the support 20 is in contact with a patient's limb, at a certain distance from the compression area 24. The contact of the support 20 is provided only by its free end or by the foot 200 of the support 20 as presented in this embodiment. That way, the device 1 does not compress other major blood vessels, which are in the vicinity of the blood vessel undergoing the medical procedure. Thus, the distance between the compression area 24 and the supporting foot 200 of the support 20 provides the space without compression that results in an undisturbed blood flow in other blood vessels that are not intended to be compressed in the medical procedure. Therefore, the contact of the device 1 with the surface of the palm side of the patient's forearm is limited to the minimum, i.e. to the compression area 24 and the free end of the support 20 or the foot 200 if it is present at the free end of the support 20, thus decreasing the discomfort level in a patient when device 1 is applied.

Despite its limited contact to the patient's limb, the support 20 provides an additional support to the device 1. The function of support 20 is, therefore, to stabilize the hollow body 2 on a patient's limb and consequently prevent any undesired movement of the device 1 after it is secured to the patient's limb. Without the support 20, the device would be prone to tipping sideways due to the forces that are applied by the strap 4. Preferably, the support 20 has a wave-type shape, as shown in Fig. 3a and 3b. Other shapes of the support 20 are also envisaged, however the wave-type shape is most effective and meets both the medical and aesthetic requirements. Additionally, the free end of the support 20 or the supporting foot 200 is provided with an inset, preferably made of an elastic material, that is applied to a surface of the supporting foot 200 or to the surface of the support 20, which comes into the direct contact with the patient's limb. This also aims to increase the patient's comfort when the device 1 is applied.

In the presently described embodiment, the hollow body 2, which is in a form of the tube, includes the side walls 22, which extend from base 26 circumferentially and are connected at the top by the externally-threaded element 21. Inside of the tube, between the base 26, side walls 22 and externally-threaded element 21 (i.e. within a periphery formed by the side walls 22, base 26 and externally-threaded element 21), there is a tubular space 23. The tubular space 23 is designed to receive a finger or a thumb of a medical personnel who, upon application of the device onto the patient's limb, puts initial compression onto the compression area 24 by pressing the base 26 of the hollow body 2. In order to provide a better access to the base 26 of the hollow body 2, the width of the side walls 22 decreases as they extend upwards (i.e. the walls gradually become narrower). The hollow body 2 is designed in a manner that the tubular space 23 can be accessed from both sides of the hollow body 2 (i.e. the tube forming the hollow body 2 is opened on both endings). This way the device is suitable to be used on both left and right forearms of the patient. This ensures a universal character of the device.

The side walls 22 provide support for a top part of a hollow body 2 - the externally-threaded element 21, which forms an integral part of the hollow body 2. In the present embodiment, the externally-threaded element 21 has a shape of a hollow screw with the axis of rotation positioned perpendicularly to an axis of the tube of the hollow body 2. The axis of rotation of the externally-threaded element 21 is also perpendicular to the surface of the base 26 of the hollow body 2. This threaded element 21 has a plurality of grooves referred to as guiding slots 27, which extend in parallel to the rotation axis of the externally-threaded element 21. In this embodiment, there are four guiding slots 27 located symmetrically on the externally-threaded element 21. The guiding slots 27 are situated along the axis of rotation and perpendicularly to the thread of the externally-threaded element 21. When the device 1 is assembled, the guiding slots 27 receive the knob supporting elements 301 and guiding elements 302 of the tightener 30. This way the hollow body 2 and the tightener 30 are engaged with each other. Two of the guiding slots 27, which are located opposite to each other and receive the guiding elements 302 of the tightener 30, are provided at the top with blocking protrusions 270. As the tightener 30 moves upwards, these blocking protrusions 270 engage with free ends of the guiding elements 302 of the tightener 30, thus blocking their further upward movement and preventing the tightener 30 from becoming disengaged from the hollow body 2. The other pair of guiding slots 27, which are also located opposite each other, receives knob supporting elements 301 of the tightener 30.

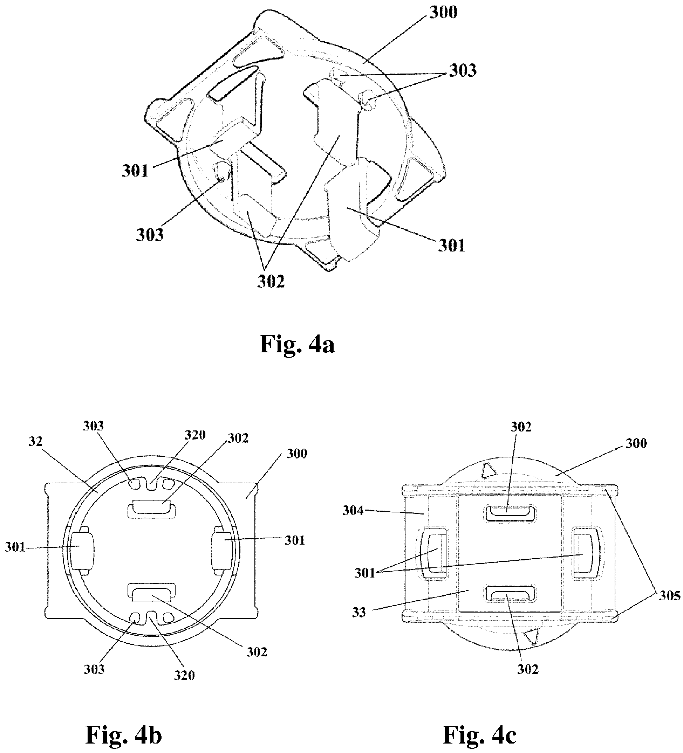

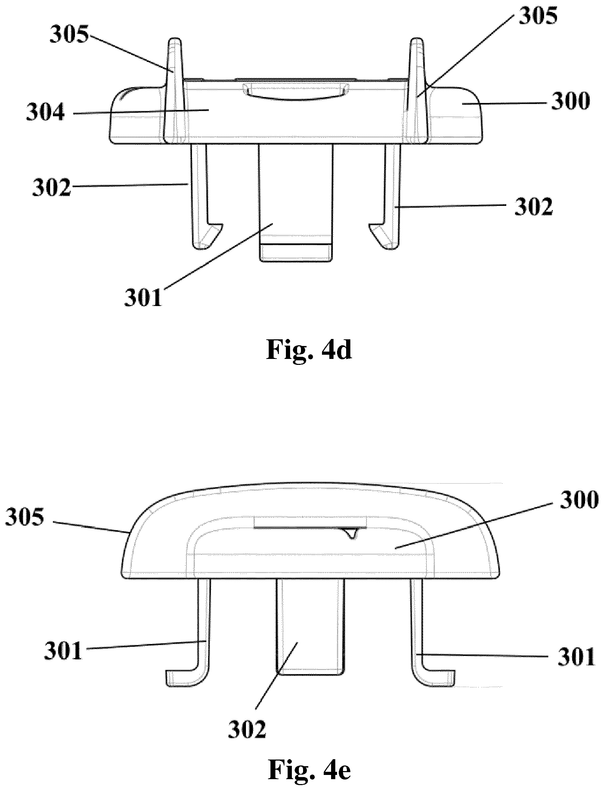

The hollow body 2 in the assembled form of the device 1 engages with the tightener 30, which is placed above the hollow body 2. Fig. 4 presents a preferred embodiment of the tightener 30 of the device of the invention. The tightener 30 comprises a housing 300, knob supporting elements 301, guiding elements 302 and a strap guide 304. The tightener 30 is located at the top of the compression device 1 (i.e. on the opposite side from the patient's limb). The tightener 30 serves as means for compression regulation of the device 1 by adjusting tension to the strap 4, when the strap is placed around the hollow body 2 and the patient's limb. The strap guide 304 is formed on the top of the tightener 30 as a support for receiving the strap 4. The strap guide 304 has a longitudinal part formed as a groove for receiving a part of the strap 4, preferably within longitudinal ribs 305 separated from each other by a distance equal to the width of the received part of the strap 4. Within the strap guide 304, in the area between the longitudinal ribs 305, an anti-skid inset 33 is placed. It is formed as a piece of sheet of plastic material, more preferably of a transparent plastic material, whose purpose is to secure the received part of the strap 4, as shown in Fig. 1a. The anti-skid inset 33 is also provided with symbols, such as "+" and "-" and arrows, as presented in Fig. 1a, printed directly on the anti-skid inset 33 or on a separate sticker placed on the anti-skid inset 33, to provide a medical personnel with information about direction, in which the knob 31 needs to be turned in order to increase or decrease the compression of the device 1.

The anti-skid inset 33 engages with the strap 4, which extends around the patient's limb and is guided over the hollow body 2 and tightener 30, between the longitudinal ribs 305 of the strap guide 304. The strap 4 extends in the direction perpendicular to the axis of the tube of the hollow body 2 and is led above and across of the hollow body 2, in a way that does not obstruct access to the tubular space 23 inside the hollow body 2. According to the preferred embodiment, as shown in Fig. 4, the strap guide 304 of the tightener 30 is shaped as a groove with two parallel ribs 305 set apart from each other. The ribs 305 have a shape of an arch and are aligned in parallel to each other. Beneath the strap guide 304, there is an integrally connected housing 300. This housing 300 has a shape of a flattened dome with a circular base. The bottom edge of the housing 300 is aligned with the ribs 305 of the strap guide 304. The housing 300 provides a covered area, which receives a ring spring 32. From the underneath of the dome of the housing 300 two knob supporting elements 301 and two guiding elements 302, protrude downwards. These elements are arranged in pairs of two supporting 301 and two guiding 302 elements, which are placed opposite to each other. They are spaced symmetrically, relative to the perimeter of the circular base of the housing 300 and each other. In this embodiment the supporting 301 and guiding 302 elements are placed away from the rim of the circular housing 300 and their spatial arrangement corresponds to the arrangement to the guiding slots 27 of the hollow body 2. These elements (i.e. supporting 301 and guiding 302 elements) slide into the guiding slots 27 of the externally-threaded element 21 of hollow body 2 when the device 1 is assembled thus providing engagement of these components, but also ensuring reciprocating movement without rotation of the tightener 30 in relation to hollow body 2. Said supporting 301 and guiding 302 elements are preferably formed as protruding beams or cuboids extending from the inside of the housing 300 dome (from the top part of the dome). The supporting 301 and guiding 302 elements at their free end (i.e. opposite to the housing 300 to which they are attached) are bent to form a foot-type support. The foot-type support of the knob supporting elements 301 are bent away from the middle axis of the housing 300, to ensure engagement of this foot-type support with the bottom edge of the knob 31, affecting the movement of the tightener 30 along with the knob 31. The foot-type support of the guiding elements 302 are bent toward the middle axis of the housing 300, to ensure engagement of this foot-type support with the blocking protrusion 270 of the guiding slot 27 of the externally-threaded element 21 of the hollow body 2. The housing 300 is also provided with studs 303 in the form of protrusions, which extend downward from the top part of the dome of the housing 300. They are designed to hold a ring spring 32 if it is present in the assembled device 1.

The guiding elements 302, not only guide the tightener 30 in reciprocating movement without rotation, but also limit the tightener 30 movement in the predefined range, by stopping the movement, when the bottom parts of the guiding elements 302 reach the top rim of the of the externally-threaded element 21 of the hollow body 2. The knob supporting elements 301 not only guide the tightener 30 in reciprocating movement without rotation, but also move the tightener 30 together with the knob 31 that is turned along the thread of the externally-threaded element 21, especially in the downward direction. During the upward movement, the knob 31 raises the tightener 30, as the tightener 30 is paced on the top of the knob 31.

All the elements of the tightener 30 - the housing 300, supporting 301 and guiding 302 elements, and a strap guide 304 - are formed as an integral element from plastic material. Preferably, the plastic material is transparent, which renders this element very aesthetic, but also is useful in the medical application of this component, as it does not obstruct the view of the patient's limb, in particular the vessel puncture site. In the most preferred embodiment the tightener 30 is made of polycarbonate by injection moulding. Thanks to its construction, the tightener 30 is easily released from the injection mould.

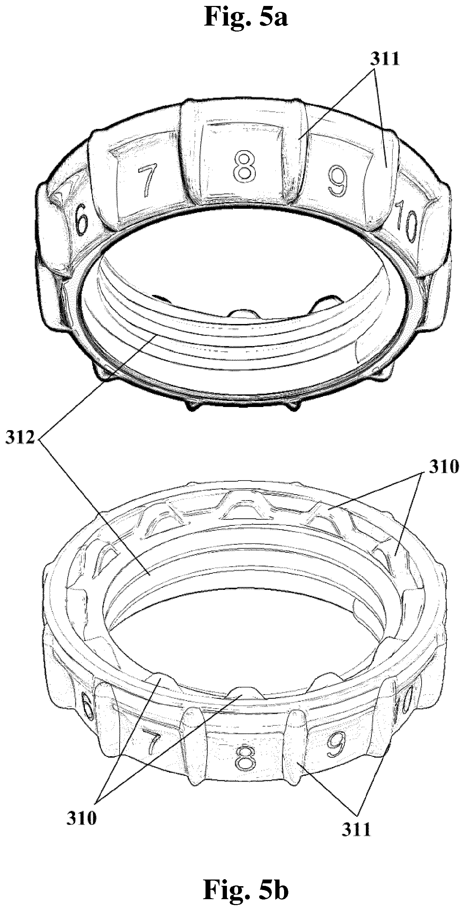

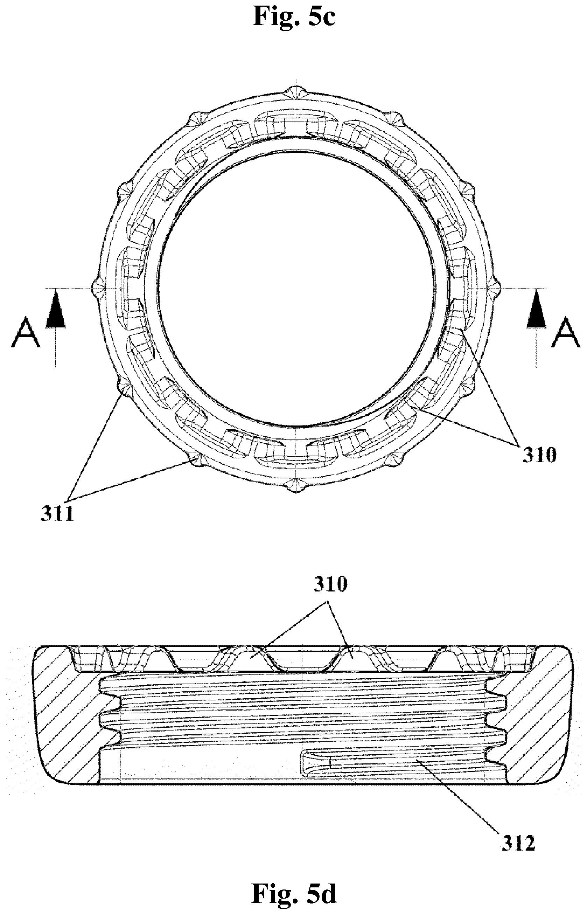

The movement of the tightener 30, and thus the compression control of the device 1, is possible due to a system comprising both the tightener 30 and the knob 31. The knob 31 as presented in Fig. 5 is preferably formed as a rotating nut whose external diameter at one end corresponds to that of the diameter of the circular base of the dome of the housing 300. The knob 31 comprises an internal opening, whose surface is provided with the internal thread 312, which engages with the thread of the externally-threaded element 21 of the hollow body 2 when the device is assembled. The external wall of the knob 31 is covered by circumferentially equally arranged pointers 311. The pointers 311 indicate the value assigned to the rotations of the knob 31. The numerical values can also be indicated between the pointers to provide additional information regarding the compression force of the device 1. The knob 31 is movably engaged with the tightener 30 through the protruding knob supporting elements 301, as it was already described. The knob 31 is placed in such way that all of the knob supporting 301 and guiding 302 elements are situated within the opening of the knob 31. In addition, the knob supporting elements 301 prevent the knob 31 against disengaging from the tightener 30, whereas the guiding elements 302 prevent both the tightener 30 and the knob 31 from slipping off the hollow body 2, by engaging with blocking protrusions 270 of the guiding slots 27 of the externally-threaded element of the hollow body 2.

The knob 31 performs both rotating and reciprocating movement, wherein the reciprocating movement of the knob 31 causes the reciprocating movement of the tightener 30. This reciprocating movement is a movement of the compression control mechanism 3, which adjusts the compression force of the device 1 onto the patient's limb. The rotating movement of the knob 31 by engagement of the external thread of the externally-threaded element 21 of the hollow body 2, onto which the knob 31 is screwed with its internal thread 312, enforces reciprocating movement of the compression control mechanism 3 perpendicularly to the limb. Such movement of the compression control mechanism 3 adjusts the strap 4 compression on the device 1, thus regulating the compression of the device 1 onto patient's limb. Further, the knob 31 in its top area has a peripherally situated groove. This grove is provided with cogs 310, which preferably are formed in a way of gearwheel teeth, i.e. the cogs 310 are protrusions, which are separated by recesses. The cogs 310 interact with the ring spring 32 placed within the housing 300 of the tightener 30 and upon rotation of the knob 31 produce the clicking sound. This clicking sound is an audio confirmation that the rotation of the knob 31 takes place, which further corresponds to the information regarding the compression of the device 1.

Similarly to the other elements of the device 1 all edges of the knob 31 are rounded not only for aesthetic purposes, but also to prevent any injury to the patient. Moreover, according to preferred embodiment, the knob 31 is formed from plastic material. More preferably, it is formed by injection moulding from a transparent plastic material, such as poly(ethylene terephthalate). The transparency of this element is advantageous for the same reasons as already described for other components of the device 1.

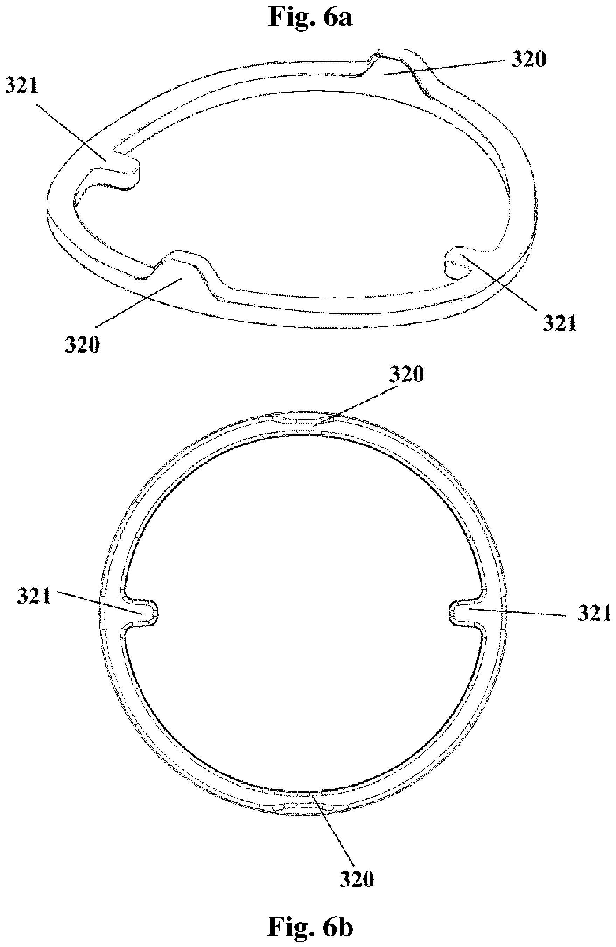

The radial compression device 1 is provided with a compression regulation element consisting of the tightener 30 and the knob 31. In accordance with the preferred embodiment of the invention the device 1 is provided with means for indicating of the displacement of the tightener 30 and the knob 31 to facilitate the compression control. Apart from the pointers 311 and value indicators located on the knob 31, in the preferred embodiment of the invention, the device is provided with audio means for indicating the rotation of the knob 31. For that purpose a ring spring 32 is provided. The ring spring 32, as shown in Fig. 6, has a shape of a ring that comprises two sets of projections - protrusions 320 and blocking tongues 321. The protrusions 320 are situated peripherally along the ring, opposite to each other. Each protrusion 320 extends in perpendicular direction to the plane containing the ring. The protrusions 320 of the ring spring 32 are engaged with recesses between the cogs 310 of the knob 31. This engagement provides desired audible clicking when the knob 31 is turned in either direction. When the knob is turned, regardless of the direction or speed of the turn, the protrusion 320 is displaced from one recess on the knob 31 to another upon which a clicking sound is made. The second set of projections - the blocking tongues 321 - prevent the ring spring 32 from rotating, when the knob 31 is turned. The blocking tongues 321 are arranged peripherally along the inner diameter of the ring. In the present embodiment they are situated within the plane of the ring and extend toward the middle of the circle defined by the ring spring 32. As shown in Fig. 4b the ring spring 32 is placed within the housing 300 of the tightener 30, between the latter and the knob 31, engaging with both by the two sets of projections 320, 321. The protrusions 320 engage with knob 31 in the recesses between the cogs 310, whereas the blocking tongues 321 are placed between studs 303, which protrude downward within the housing 300 of the tightener 30. The distance between two adjacent studs 303 corresponds to the width of the corresponding blocking tongue 321.

In the assembled device 1, the ring spring 32 is placed within the dome of the housing 300, so that it is adjacent to the surrounding walls of the housing 300, the blocking tongues 321 are inserted between the adjacent studs 303 of the housing 300, and the protrusions 320 extent downward towards the recesses between the cogs 310 of the knob 31. In the preferred embodiment the ring spring 32 is bent, so that its parts equipped with protrusions 320 project from the plane of the spring ring 32 in the direction of the knob 31. Moreover, the ring spring 32 is formed from plastic material. In the preferred embodiment the plastic material is elastic to ensure the desired flexibility of the ring spring 32. In the most preferred embodiment the ring spring 32 is made of polyetherimide, which provides both flexibility and durability of the ring spring 32. The use of the ring spring 32 is very advantageous because it provides the clicking mechanism and, at the same time, does not increase the dimensions of the device 1 (i.e. the clicking mechanism has been significantly miniaturized).

As described above, the movement of aforesaid compression control mechanism 3, which comprises the tightener 30 and the knob 31, is responsible for tightening or loosen the strap 4 extending around the patient's limb and the tightener 30. This way, the device 1 provides easy control of the compression force applied to blood vessels in the limb. While the knob 31 is rotated, it provides reciprocating movement of the compression control mechanism 3 in a direction perpendicular to the surface of the limb. Upon upward movement of the tightener 30, the increasing tension is applied to a strap 4, which in turns generates the increased compression of the hollow body 2 onto the patient's limb.

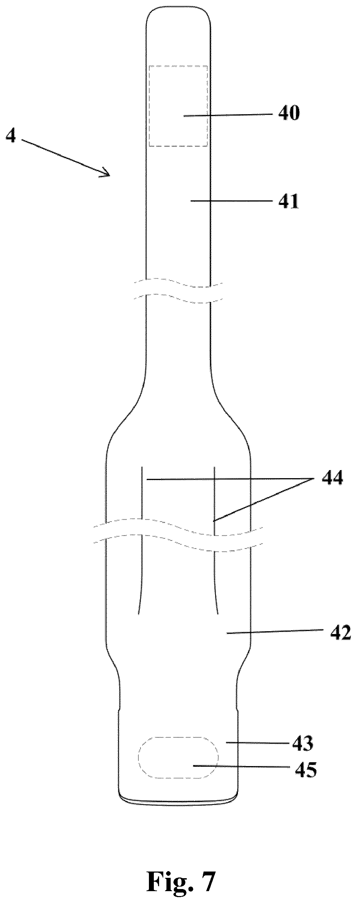

The strap 4 is an integral element, i.e. it is formed from a single piece of a plastic material. In the preferred embodiment the strap 4 has also anti-slip properties, i.e. the surfaces thereof when in contact do not slide against each other. This also increases the stability of the device 1, when it is applied to the patient's limb. Preferably, the strap 4 is transparent, i.e. it does not obstruct the view of the patient's limb. At the same time, the strap 4 is elastic, it can be wrapped around the patient's limb and over the top of the device 1. As shown in Fig. 7, the strap 6 can be divided into three sections, which pass one into another: a narrow strap part 41, which starts at the free end of the strap 4, a wide strap part 42, which forms the middle part of the strap 4, and a connection strap part 43, which provides attachment of the strap 4 to the hollow body 2. When the device 1 is applied to a patient's limb the narrow strap part 41 extends over the tightener 30 and is wrapped around the patient's limb again. Therefore the width of the narrow strap part 41 corresponds to the width of the strap guide 304 of the tightener 30. The narrow strap part 41 is optionally provided with a locking sticker 40 in the vicinity of the free end of the strap 4, which helps to secure the strap 4 in the folded position.

The narrow strap part 41 extents, preferably gradually, into the wide strap part 42. When the device is applied into the patent's limb the wide strap part 42 is in contact with the dorsal side of patient's limb. The increased width of the strap 4 in this section increases patient's comfort and stability of the device when it is applied to the limb. The wide strap part 42 is preferably provided with two guiding edges 44, which extend in the lengthwise direction of the strap 4 and form a groove. The distance between the guiding edges 44 corresponds to the width of the narrow strap part 41, which is received in the groove formed between the guiding edges 44, when the device 1 is applied to the limb. The guiding edges 44 ensure that the narrow strap part 41 is held in place when the strap 4 is wrapped around the patient's limb.

The strap 4 is connected to the hollow body 2 by the connection strap part 43. The connection between the strap 4 and the hollow body 2 is durable, since it withstands forces associated with tension generated by upward movement of the tightener 30. In the present embodiment, the connection strap part 43 is connected to the hollow body 2 by an adhesive 5 that is placed between the connection strap part 43 and the compression area 24 of the hollow body 2. In addition, on the surface opposite to the adhesive 5, the connection strap part 43 is provided with the compression inset 45 (i.e. the compression inset 45 faces the limb of the patient). The compression inset 45 is preferably formed from an elastic material and provides additional compression in desired area of the limb.

The vessel compression device 1 according to the preferred embodiment, as shown in Fig. 1a and 1b, is applied to a limb by placing the main compression area 24 of the hollow body 2 onto the appropriate blood vessel that requires the compression. Regardless of the vessel compressed or the limb onto which the device of the invention is mounted, the stabilizing support 20, that provides proper support and securing of the position of the device, always faces the middle of the wrist. For example, if the device is attached to a left wrist and radial artery is to be compressed, the support 20 protrudes in the direction of the wrist middle. The device is applied to the limb by pressing the hollow body 2 by means of tubular space 23 of the hollow body 2 (e.g. by pressing the inner surface of the base 26 of the tube), and is secured and fastened by wrapping the strap 4 around the limb and also around the device 1 itself. The end of strap 4 is secured, so that unwinding of the strap 4 is prevented. Preferably, the end of the strap 4 is secured by the adhesive, such as the locking sticker 40. That way any accidental releasing of the compression is also prevented.

### List of elements:

1 - a vessel compression device
2 - a hollow body / tube
20 - a support
200 - a supporting foot of the support
21 - an externally-threaded element
22 - a side wall of the hollow body
23 - a tubular space
24 - a compression area (i.e. the outer surface of the longitudinal base)
25 - a positioning mark
26 - a longitudinal base
27 - a guiding slot
270 - a blocking protrusion
3 - a compression control mechanism
30 - a tightener
300 - a housing
301 - a knob supporting element
302 - a guiding element
303 - a stud
304 - a strap guide
305 - longitudinal ribs of the strap guide
31 - a knob
310 - a cog
311 - a pointer
312 - a internal thread
32 - a ring spring
320 - a protrusion
321 - a blocking tongue
33 - an anti-skid inset
4 - a strap
40 - a locking sticker at the free end of the strap
41 - a narrow strap part
42 - a wide strap part
43 - a connection strap part
44 - guiding edges
45 - a main compression inset
5 - an adhesive for securing the strap to the hollow body

## Claims

1. A blood vessel compression device for a limb comprising:
a hollow body (2) having an internal substantially annular surface and a longitudinal base (26) defining a generally tubular space (23) extending in a longitudinal direction, said base being accessible by the generally tubular space (23);
a compression area (24) located at an external surface of the base (26);
a compression control mechanism (3) for regulating compression applied by the device, ;
a strap (4) for securing the device to the limb and effecting compression thereon, the strap being attached to body (2) at the base (26) and releasably attachable with and adjustable by the compression control mechanism (3) without blocking the tubular space (23), **characterized in that** the compression control mechanism (3) is located on the body (2) distal to and opposite the compression area (24) and the base (26) and the device further comprises a support (20) for stabilising the device on the limb, the support being located adjacent the base (26) and extending in a longitudinal direction parallel therewith.

2. The device of claim 1, wherein the longitudinal base (26) is a flattened surface that partly forms the internal substantially annular surface of the body (2).

3. The device of claim 1 or 2, wherein the compression control mechanism (3) is a screw and nut mechanism.

4. The device of claim 3, wherein the screw-and-nut mechanism comprises
- an externally-threaded element (21) in a form of a screw with the rotation axis perpendicular to the compression area (24), which is provided with a plurality of thread-cutting guiding slots (27) that are parallel to the rotation axis of said externally-threaded element (21),
- a knob (31) in a nut-like form, which is fitted over the externally-threaded element (21) in an engaging manner, and
- a tightener (30), which is positioned on the knob (31), wherein the tightener (30) comprises a housing (300) and the knob supporting (301) and guiding (302) elements, which protrude from the housing (300) in the direction of and perpendicularly to the compression area (24), wherein said knob supporting (301) and guiding (302) elements are received by the guiding slots (27) of the externally-threaded element (21).

5. The device of claim 4, wherein the externally-threaded element (21) is provided with four guiding slots (27) positioned symmetrically on the externally-threaded element (21), and the tightener (30) is provided with two corresponding pairs of the knob supporting (301) and guiding (302) elements, and wherein each pair of the knob supporting (301) and guiding (302) elements is received by a pair of guiding slots (27) positioned opposite to each other.

6. The device of anyone of claims 1 to 5, wherein the support (20) is in a form of a bent fin of a wave-type cross section, which at the free end thereof is provided with a foot (200), wherein only the foot (200) of the support (20) is in the same plane as the compression area (24).

7. The device of any one of claims 1 to 6, wherein the compression control mechanism (3) comprises a clicking sound producing means that is configured to emit an audible click upon adjustment of the compression control mechanism (3), wherein the clicking sound producing means comprise
- a ring spring (32), which is fixed within the housing (300) of the tightener (30) and has at least one protrusion (320) that extends towards the knob (31), and
- cogs (310) uniformly spaced along the rim of the knob (31) in the proximity of the tightener (30), so that upon rotation of the knob (31) the protrusion (320) engages with one of the cogs (310) thereby producing a clicking sound.

8. The device of anyone of claim 1 to 7, wherein the strap (4) is attached to the hollow body (2) at the compression area (24) by the means of an adhesive (5), wherein the attachment is formed over the entire compression area (24).

9. The device of anyone of claims 1 to 8, wherein the compression area (24) and the foot (200) of the support (20) are provided with elastic insets.

10. The device of anyone of claims 1 to 9, wherein all of the device components are made of transparent plastic material.

## Patentansprüche

1. Blutgefäßkompressionsvorrichtung für eine Gliedmaße, umfassend:
einen Hohlkörper (2), der eine im Wesentlichen ringförmige innere Fläche und eine längliche Basis (26) aufweist, die einen im Allgemeinen röhrenförmigen Raum (23) definiert, der sich in einer Längsrichtung erstreckt, wobei die Basis durch den im Allgemeinen röhrenförmigen Raum (23) zugänglich ist;
einen Kompressionsbereich (24), der sich an einer Außenfläche der Basis (26) befindet;
einen Kompressionssteuermechanismus (3) zum Regulieren der Kompression, die durch die Vorrichtung ausgeübt wird;
einen Gurt (4) zum Befestigen der Vorrichtung an der Gliedmaße und Bewirken von Kompression darauf, wobei der Gurt an dem Körper (2) an der Basis (26) angebracht ist und lösbar an dem Kompressionssteuermechanismus (3) anbringbar und durch diesen anpassbar ist, ohne den röhrenförmigen Raum (23) zu blockieren, **dadurch gekennzeichnet, dass** der Kompressionssteuermechanismus (3) an dem Körper (2) distal zu und entgegengesetzt dem Kompressionsbereich (24) und der Basis (26) angeordnet ist und die Vorrichtung ferner eine Stütze (20) zum Stabilisieren der Vorrichtung an der Gliedmaße umfasst, wobei sich die Stütze neben der Basis (26) befindet und sich damit in einer Längsrichtung parallel erstreckt.

2. Vorrichtung nach Anspruch 1, wobei die längliche Basis (26) eine abgeflachte Fläche ist, die teilweise die im Wesentlichen ringförmige innere Fläche des Körpers (2) bildet.

3. Vorrichtung nach Anspruch 1 oder 2, wobei der Kompressionssteuermechanismus (3) ein Schrauben-Mutter-Mechanismus ist.

4. Vorrichtung nach Anspruch 3, wobei der Schrauben-Mutter-Mechanismus umfasst:
- ein Außengewindeelement (21) in Form einer Schraube mit der Drehachse senkrecht zu dem Kompressionsbereich (24), das mit mehreren Gewindeschneidführungsschlitzen (27) versehen ist, die parallel zu der Drehachse des Außengewindeelements (21) verlaufen,
- einen Knopf (31) in einer mutterartigen Form, der eingreifend über dem Außengewindeelement (21) angebracht ist, und
- eine Spannvorrichtung (30), die auf dem Knopf (31) angeordnet ist, wobei die Spannvorrichtung (30) ein Gehäuse (300) und die Elemente zum Stützen (301) und Führen (302) des Knopfes umfasst, die in Richtung des Kompressionsbereichs (24) und senkrecht dazu von dem Gehäuse (300) hervorstehen, wobei die Elemente zum Stützen (301) und Führen (302) des Knopfes von den Führungsschlitzen (27) des Außengewindeelements (21) aufgenommen sind.

5. Vorrichtung nach Anspruch 4, wobei das Außengewindeelement (21) mit vier Führungsschlitzen (27) versehen ist, die an dem Außengewindeelement (21) symmetrisch angeordnet sind, und die Spannvorrichtung (30) mit zwei entsprechenden Paaren der Elemente zum Stützen (301) und Führen (302) des Knopfes versehen ist, und wobei jedes Paar der Elemente zum Stützen (301) und Führen (302) des Knopfes von einem Paar von Führungsschlitzen (27) aufgenommen ist, die einander entgegengesetzt angeordnet sind.

6. Vorrichtung nach einem der Ansprüche 1 bis 5, wobei die Stütze (20) die Form einer gebogenen Rippe mit einem wellenförmigen Querschnitt aufweist, die an ihrem freien Ende mit einem Fuß (200) versehen ist, und wobei sich nur der Fuß (200) der Stütze (20) in der gleichen Ebene wie der Kompressionsbereich (24) befindet.

7. Vorrichtung nach einem der Ansprüche 1 bis 6, wobei der Kompressionssteuermechanismus (3) ein Klickgeräuscherzeugungsmittel umfasst, das konfiguriert ist, beim Anpassen des Kompressionssteuermechanismus (3) ein hörbares Klicken zu erzeugen, wobei das Klickgeräuscherzeugungsmittel
- eine Ringfeder (32), die innerhalb des Gehäuses (300) der Spannvorrichtung (30) befestigt ist und mindestens einen Vorsprung (320) aufweist, der sich in Richtung des Knopfes (31) erstreckt, und
- Zähne (310) umfasst, die entlang des Randes des Knopfes (31) in der Nähe der Spannvorrichtung (30) gleichmäßig beabstandet sind, sodass bei einer Drehung des Knopfes (31) der Vorsprung (320) in einen der Zähne (310) eingreift und dadurch ein Klickgeräusch erzeugt wird.

8. Vorrichtung nach einem der Ansprüche 1 bis 7, wobei der Gurt (4) mittels eines Haftmittels (5) an dem Hohlkörper (2) an dem Kompressionsbereich (24) angebracht ist, und wobei die Anbringung über den gesamten Kompressionsbereich (24) gebildet ist.

9. Vorrichtung nach einem der Ansprüche 1 bis 8, wobei der Kompressionsbereich (24) und der Fuß (200) der Stütze (20) mit elastischen Einsätzen versehen sind.

10. Vorrichtung nach einem der Ansprüche 1 bis 9, wobei alle Vorrichtungskomponenten aus transparentem Kunststoffmaterial hergestellt sind.

## Revendications

1. Dispositif de compression de vaisseau sanguin pour un membre comprenant :
un corps creux (2) ayant une surface interne sensiblement annulaire et une base longitudinale (26) définissant un espace généralement tubulaire (23) s'étendant dans une direction longitudinale,
ladite base étant accessible par l'espace généralement tubulaire (23) ;
une zone de compression (24) située sur une surface externe de la base (26) ;
un mécanisme de commande de compression (3) pour la régulation de la compression appliquée par le dispositif ;
une bande (4) pour la fixation du dispositif au membre et la réalisation d'une compression sur celui-ci, la bande étant fixée au corps (2) sur la base (26) et pouvant être fixée de manière libérable avec et réglable par le mécanisme de commande de compression (3) sans blocage de l'espace tubulaire (23), **caractérisé en ce que** le mécanisme de commande de compression (3) est situé sur le corps (2) de manière distale par rapport à et opposée à la zone de compression (24) et à la base (26) et le dispositif comprend en outre un support (20) pour la stabilisation du dispositif sur le membre,
le support étant situé adjacent à la base (26) et s'étendant dans une direction longitudinale parallèle à celle-ci.

2. Dispositif selon la revendication 1, dans lequel la base longitudinale (26) est une surface aplatie qui forme partiellement la surface sensiblement annulaire interne du corps (2).

3. Dispositif selon la revendication 1 ou 2, dans lequel le mécanisme de commande de compression (3) est un mécanisme à vis et écrou.

4. Dispositif selon la revendication 3, dans lequel le mécanisme à vis et écrou comprend
- un élément fileté extérieurement (21) en forme de vis avec l'axe de rotation perpendiculaire à la zone de compression (24), qui est fournie avec une pluralité de fentes de guidage de filetage (27) qui sont parallèles à l'axe de rotation dudit élément fileté extérieurement (21),
- un bouton (31) en forme d'écrou, qui est ajusté sur l'élément fileté extérieurement (21) en une manière d'engagement, et
- un dispositif de serrage (30), qui est positionné sur le bouton (31), dans lequel le dispositif de serrage (30) comprend un logement (300) et les éléments de support (301) et de guidage (302) du bouton, qui font saillie du logement (300) dans la direction de et perpendiculairement à la zone de compression (24), dans lequel lesdits éléments de support (301) et de guidage (302) du bouton sont reçus par les fentes de guidage (27) de l'élément fileté extérieurement (21).

5. Dispositif selon la revendication 4, dans lequel l'élément fileté extérieurement (21) est fourni avec quatre fentes de guidage (27) positionnées symétriquement sur l'élément fileté extérieurement (21), et le dispositif de serrage (30) est fourni avec deux paires correspondantes d'éléments de support (301) et de guidage (302) de bouton, et dans lequel chaque paire d'éléments de support (301) et de guidage (302) de bouton est reçue par une paire de fentes de guidage (27) positionnées à l'opposé l'une de l'autre.

6. Dispositif selon l'une quelconque des revendications 1 à 5, dans lequel le support (20) est sous la forme d'une ailette courbée ayant une section transversale de type ondulé, qui est fournie à son extrémité libre avec un pied (200), dans lequel seul le pied (200) du support (20) est dans le même plan que la zone de compression (24).

7. Dispositif selon l'une quelconque des revendications 1 à 6, dans lequel le mécanisme de commande de compression (3) comprend un moyen de production de clic sonore qui est configuré pour émettre un clic audible lors du réglage du mécanisme de commande de compression (3), dans lequel le moyen de production de clic sonore comprend
- un ressort annulaire (32), qui est fixé à l'intérieur du logement (300) du dispositif de serrage (30) et a au moins une saillie (320) qui s'étend vers le bouton (31), et
- des crans (310) espacés uniformément le long du bord du bouton (31) à proximité du dispositif de serrage (30), de sorte que lors de la rotation du bouton (31), la saillie (320) s'engage avec un des crans (310) produisant ainsi un clic sonore.

8. Dispositif selon l'une quelconque des revendications 1 à 7, dans lequel la bande (4) est fixée au corps creux (2) sur la zone de compression (24) au moyen d'un adhésif (5), dans lequel la fixation est formée sur toute la zone de compression (24).

9. Dispositif selon l'une quelconque des revendications 1 à 8, dans lequel la zone de compression (24) et le pied (200) du support (20) sont fournis avec des inserts élastiques.

10. Dispositif selon l'une quelconque des revendications 1 à 9, dans lequel tous les composants du dispositif sont constitués d'une matière plastique transparente.
